(19) Europäisches Patentamt
European Patent Office
Office européen des brevets

(11) Veröffentlichungsnummer: **0 121 773**
**B1**

(12) # EUROPÄISCHE PATENTSCHRIFT

(45) Veröffentlichungstag der Patentschrift: **09.09.87**

(51) Int. Cl.⁴: **C 07 D311/72**

(21) Anmeldenummer: **84102435.9**

(22) Anmeldetag: **07.03.84**

(54) **Verbessertes Verfahren zur Herstellung von alpha-Tocopherol.**

(30) Priorität: **15.03.83 DE 3309158**

(43) Veröffentlichungstag der Anmeldung: **17.10.84 Patentblatt 84/42**

(45) Bekanntmachung des Hinweises auf die Patenterteilung: **09.09.87 Patentblatt 87/37**

(84) Benannte Vertragsstaaten: **CH DE FR GB LI NL**

(56) Entgegenhaltungen: **DE-A- 2 602 509**

(73) Patentinhaber: **BASF Aktiengesellschaft**
**Carl-Bosch-Strasse 38**
**D-6700 Ludwigshafen (DE)**

(72) Erfinder: **Ernst, Hansgeorg, Dr.**
**Bruesseler Ring 38**
**D-6700 Ludwigshafen (DE)**
Erfinder: **Gehrken, Henning-Peter, Dr.**
**Wiesenweg 6**
**D-6715 Lambsheim (DE)**
Erfinder: **Paust, Joachim, Dr.**
**Ringstrasse 3**
**D-6708 Neuhofen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein verbessertes Verfahren zur Herstellung von α-Tocopherol durch Umsetzung eines Chromanderivates mit einem $C_{14}$-Grignard-Reagens in Gegenwart eines Dialkalimetallhalogencuprats, vorzugsweise Dilithiumtetrachlorocuprat. Dieses Verfahren ermöglicht neben der Herstellung von racemischem α-Tocopherol die vorteilhafte Herstellung von natürlich konfiguriertem (2R, 4'R, 8'R)-α-Tocopherol, sowie von (2R, 4'RS, 8'RS)-α-Tocopherol oder beliebigen anderen α-Tocopherolstereoisomeren.

In den vergangenen Jahren hat Vitamin E (α-Tocopherol) als Antioxidans sowie auf dem Gebiet der menschlichen und tierischen Ernährung Bedeutung erlangt. Zur Synthese von all-rac-α-Tocopherol sind die verschiedensten Verfahren bekannt. Eine Übersichtsarbeit von Siebrell und Harris hierüber findet sich in « Vitamins », Vol V, Seiten 165 f. (1972). In der neueren Literatur sind auch Verfahren zur Darstellung von natürlichem optisch aktivem Vitamin E (2R, 4'R, 8'R-α-Tocopherol) beschrieben worden [vgl. N. Cohen et al, J. Am. Chem. Soc. 101 (1979) Seiten 6710-16]. Die Darstellung von Stereoisomeren des α-Tocopherols ist von Interesse, weil den verschiedenen Stereoisomeren eine verschieden starke biologische Wirkung zukommt. Von den drei Asymmetriezentren des natürlichen Vitamins E der Formel

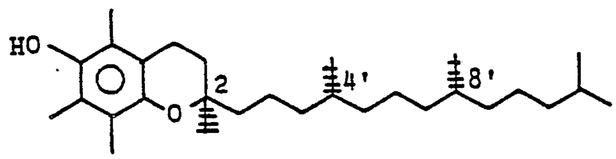

ist das Zentrum am C-Atom in 2-Stellung von besonderer Wichtigkeit für die biologische Wirkung [vgl. S. Ames, « Lipids » No. 6 (1971) Seiten 281 bis 290, insbesondere S. 285]. So zeigt z. B. das (2R, 4'RS, 8'RS) — gegenüber dem (2S, 4'RS, 8'RS) — Epimeren eine 5-fach höhere Wirksamkeit. Da die Zentren C-4' und C-8' die Wirksamkeit des Moleküls nur untergeordnet beeinflussen, ist auch eine Synthese von (2R, 4'RS, 8'RS)-α-Tocopherol bereits zur Erzielung einer erhöhten biologischen Aktivität geeignet.

Cohen et al. beschreiben in Helv. Chim. Acta, Vol. 64 Fasc. 4 (1981) S. 1158-73 die Herstellung aller 8 möglichen Stereoisomeren von α-Tocopheryl-acetat in hoher chemischer und stereoisomerer Reinheit. Das (2R, 4'RS, 8'RS)-Isomere erhielten sie beispielsweise durch Umsetzen von racem. Tetrahydronerolidol mit Triphenylphosphoniumbromid in $CH_2Cl_2$ und Wittig-Reaktion des dabei erhaltenen Triphenylphosphoniumsalzes mit (+)-(S)-6-Benzyloxy-2,5,7-tetramethylchroman-2-carbaldehyd in Gegenwart von Natriummethylat und anschließende Hydrierung des erhaltenen α-Tocodiens an Pd/C.

Die Herstellung von racemischem und optisch aktivem α-Tocopherol durch Kupplung eines entsprechenden Chromanderivates, das bereits eine $C_2$-Seitenkette in 2-Stellung aufweist mit einem geeigneten $C_{14}$-Baustein wird in der DE-OS 26 02 509 beschrieben. Gemäß dieser DE-OS wird ein Chromanderivat der Formel

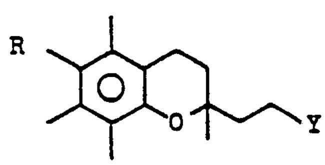

in der R eine Ethergruppe darstellt und Y eine Fluchtgruppe bedeutet, in einem organischen Lösungsmittel mit einer Grignard-Verbindung der allgemeinen Formel IV

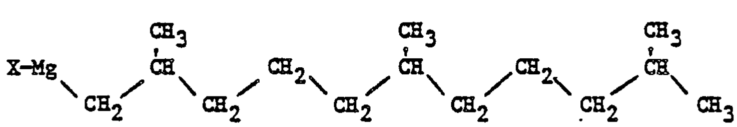

unter Katalyse mit einem Dialkalimetalltetrahalogencuprat nach Schlosser-Fouquet [vgl. Angew. Chem. Int. Ed. 13 (1974), S. 82 f und ibid. 13, Seite 701 f.] verknüpft.

Nachteilig an diesem Verfahren ist, daß nach der Kupplung zur Herstellung von α-Tocopherol die in 6-Stellung des Chromansystems vor der Kupplung zunächst eingeführte Etherschutzgruppe wieder abgespalten werden muß. Diese zusätzlichen 2 Reaktionsstufen bringen natürlich Ausbeuteverluste sowie zusätzliche Einsatzstoffkosten mit sich.

Aufgabe der Erfindung war es daher, die Herstellung von α-Tocopherol durch Verknüpfung eines eine $C_2$-Seitenkette in 2-Stellung enthaltenden Chromanderivats mit einem entsprechenden $C_{14}$-

Baustein vorteilhafter zu gestalten.

Gegenstand der Erfindung ist nun ein Verfahren zur Herstellung von α-Tocopherol der Formel I

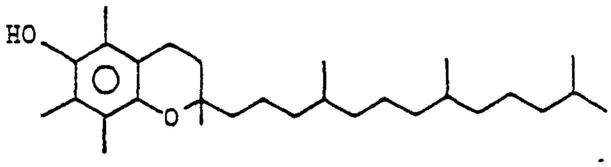

(I)

durch Umsetzung eines Chromanderivates mit einem $C_{14}$-Grignard-Reagens unter Katalyse mit einem Dialkalimetalltetrahalogencuprat nach Schlosser-Fouquet, das dadurch gekennzeichnet ist, daß man

a) ein Chromanderivat der allgemeinen Formel II

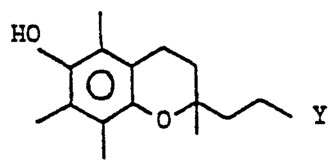

(II)

in der Y für eine der Fluchtgruppen -Br, -J, -p-Tosyl oder Benzolsulfonyl, insbesondere für -Br, steht, verwendet und dieses bei Temperaturen von —70 bis 0 °C, vorzugsweise —25 bis —15 °C

b) zunächst mit der Lösung von 0,8 bis 1,1, vorzugsweise 0,85 bis 1,0 Äquivalenten eines Grignard-Reagenses der allgemeinen Formel III

X—Mg—R (III),

in der X für Cl, Br oder J steht und R eine geradkettige oder verzweigte Alkylgruppe mit 1 bis 14 C-Atomen, vorzugsweise eine Methylgruppe, eine Ethylgruppe oder den Rest

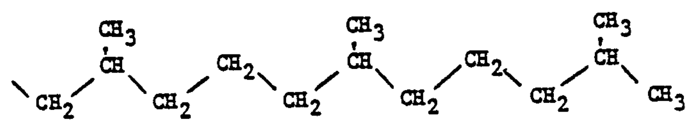

bedeutet, und dann

c) mit der Lösung von 1,05 bis 2,0 Äquivalenten, vorzugsweise 1,2 bis 1,7 Äquivalenten eines Grignard-Reagenses der Formel IIIa

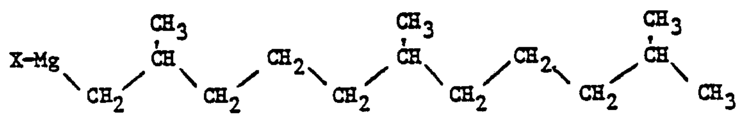

(IIIa)

in einem etherischen Lösungsmittel und in Gegenwart von Kupferionen, vorzugsweise in Gegenwart der Lösung eines Dialkalimetalltetrahalogencuprats, insbesondere von Dilithiumtetrachlorocuprat in einem etherischen Lösungsmittel umsetzt.

Besondere Bedeutung hat das erfindungsgemäße Verfahren für die Herstellung von (2R, 4'RS, 8'RS)-α-Tocopherol und dem natürlichen (2R, 4'R, 8'R)-α-Tocopherol.

Zur Herstellung dieser Verbindungen wird mit Vorteil (2S)-2,5,7,8-Tetramethyl-6-hydroxy-2-(2'-bromethyl)-chroman in der Stufe c) mit einem (2RS, 6RS)-2,6,10-Trimethyl-1-undecylmagnesiumhalogenid bzw. mit einem (2R, 6R)-2,6,10-Trimethyl-1-undecylmagnesiumhalogenid umgesetzt. Das neue Verfahren verkürzt den Syntheseweg zu α-Tocopherol der Formel I insofern, als bei der erfindungsgemäßen Umsetzung eines Chromanderivates mit einem $C_{14}$-Grignardreagens die Umsetzung ohne vorherige Einführung und dementsprechend ohne spätere Abspaltung einer Schutzgruppe erfolgen kann.

Als Chromanderivate der allgemeinen Formel II kommen diejenigen in Betracht, in denen Y eine während der Grignard-Umsetzung austretende Gruppe (sogenannte Fluchtgruppe) bedeutet. Genannt

seien folgende Fluchtgruppen wie -Br, -J, -p-Tosyl und -Benzolsulfonyl, insbesondere -Br.

Sie können in Form des Racemats oder in optisch aktiver Form eingesetzt werden.

Die Verknüpfungsreaktion wird in einem inerten etherischen Lösungsmittel, z. B. in Tetrahydrofuran (THF), Dioxan, Diethylether, Dimethoxyethan oder in Diethylenglykoldimethylether durchgeführt. Vorzugsweise verwendet man THF.

Die Grignard-Verbindungen der allgemeinen Formel III bzw. IIIa können in an sich bekannter Weise z. B. dadurch hergestellt werden, daß man die entsprechenden Alkohole in ein Halogenid überführt und dieses in einem etherischen Lösungsmittel, zweckmäßig in THF, bei erhöhter Temperatur, z. B. in einem Temperaturbereich zwischen 40 und 80 °C, mit metallischem Magnesium behandelt. Eine vorteilhafte Methode zur Herstellung von (2R, 6R)-1-Chlor-2,6,10-trimethyl-undecan wird in der DE-A 31 39 238 beschrieben.

Zur Durchführung der Kupplungsreaktion wird das Chromanderivat II in dem etherischen Lösungsmittel gelöst und die Lösung bei Temperaturen von —70 bis 0 °C, vorzugsweise —25 bis —15 °C zunächst mit 0,8 bis 1,1 Äquivalent, vorzugsweise 0,85 bis 1,0 Äquivalenten einer etwa 1 bis 2 molaren Lösung eines Grignard-Reagenses der Formel III versetzt. Hierbei kann man die später für die Kupplung einzusetzende Grignard-Verbindung IIIa, aber auch andere beliebige Alkylgrignardverbindungen einsetzen. Selbst ungesättigte Verbindungen, wie die Vinylmagnesiumhalogenide können verwendet werden, bieten jedoch keine Vorteile, da sie einerseits schwieriger herstellbar sind und bei ihrer Verwendung die Ausbeuten geringer sind.

Mit Vorteil verwendet man Methylmagnesiumbromid oder Ethylmagnesiumbromid.

Die bei diesem Reaktionsschritt verwendeten Grignard-Verbindungen dienen dazu, die phenolische OH-Gruppe der Chromanverbindung nach Art einer Zerewitinoff-Reaktion (vgl. Organikum VEB Deutscher Verlag der Wissenschaftlicher, Berlin, 1972, 11. Auflage, S. 551) zu deprotonieren und so in eine Magnesiumphenolatgruppe zu überführen.

Man läßt dazu die beiden Komponenten im angegebenen Temperaturbereich zwischen 15 min und 4 h, vorzugsweise 1/2 bis 2 h, reagieren.

Im Anschluß daran gibt man bei derselben Temperatur zum Reaktionsgemisch die Grignard-Verbindung IIIa in Form einer etwa 1 bis 2 molaren Lösung in einem etherischen Lösungsmittel zu. Hierbei verwendet man IIIa in einem kleinen molaren Überschuß (1,05 bis 2 Äquivalente, bezogen auf II, vorzugsweise 1,2 bis 1,7 Äquivalente).

Nach Zugabe des Dialkalimetalltetrahalogencuprats, das zweckmäßig in Form einer etwa 0,5 molaren Lösung in dem etherischen Lösungsmittel verwendet wird, läßt man das Reaktionsgemisch etwa 2 bis 3 h bei —70 bis 0 °C, vorzugsweise bei —25 bis —15 °C nachrühren, läßt dann das Reaktionsgemisch auf Raumtemperatur kommen und rührt nach 4 bis 20 Stunden bei Raumtemperatur nach. Als Dialkalimetalltetrahalogencuprat verwendet man vorzugsweise das Dilithiumtetrachlorocuprat. Im allgemeinen werden Mengen von etwa 0,5 bis 4 Mol.%, vorzugsweise 1 bis 3 Mol.%, bezogen auf die eingesetzte Grignard-Verbindung IIIa verwendet.

Die Aufarbeitung des Reaktionsgemisches erfolgt in üblicher Weise, beispielsweise durch Versetzen mit einer gesättigten Ammoniumchloridlösung und Extraktion mit Diethylether.

Mit Hilfe des erfindungsgemäßen Verfahrens wird die Herstellung von 2RS, 4'RS, 8'RS-, 2R, 4'RS, 8'RS- oder 2R, 4'R, 8'R-α-Tocopherol durch Umsetzen eines eine $C_2$-Seitenkette in 2-Stellung enthaltenden Chromanbausteins mit der entsprechenden $C_{14}$-Grignard-Verbindung wesentlich vereinfacht.

## Beispiel 1

a) Herstellung einer (2RS, 6RS)-2,6,10-Trimethyl-1-undecyl-magnesium-chlorid-lösung

Zu 1,48 g (61,8 mmol) Magnesiumspänen, die durch Anätzen mit einem Jodkristall aktiviert worden waren, wurden zunächst 10 ml Tetrahydrofuran (THF) und dann eine Lösung von 13,95 g (60 mmol) (2RS, 6RS)-1-Chlor-2,6,10-trimethylundecan in 20 ml THF getropft. Das Gemisch wurde 2 Stunden bei + 70 °C nachgerührt.

b) Umsetzung mit den Grignard-reagentien

15,65 g (50 mmol) (2RS)-2,5,7,8-Tetramethyl-6-hydroxy-2-(2-bromethyl)-chroman wurden in 30 ml absolutem THF gelöst, zu dieser Lösung bei — 20 °C 33,5 ml einer 1,5 molaren Lösung von Methylmagnesiumbromid in THF zugetropft und das erhaltene Reaktionsgemisch 30 Minuten bei — 20 °C nachgerührt. Anschließend wurden erst die unter a) hergestellte Lösung von (2RS, 6RS)-2,6,10-Trimethyl-1-undecylmagnesiumchlorid und dann 3,3 ml einer 0,5 molaren Lösung von Dilithiumtetrachlorocuprat zugefügt und das erhaltene Reaktionsgemisch noch 3 h bei —20 °C und dann 15 h bei Raumtemperatur nachgerührt.

c) Aufarbeitung des Reaktionsgemisches

Das gemäß b) erhaltene Reaktionsgemisch wurde mit 100 ml einer gesättigten Ammoniumchloridlösung versetzt und mit Diethylether extrahiert. Die vereinigten organischen Phasen wurden mit gesättigter Ammoniumchloridlösung und 5 %iger Natriumhydrogencarbonatlösung gewaschen, über $MgSO_4$ getrocknet und das Lösungsmittel unter vermindertem Druck abgedampft, wobei ein öliges Produkt erhalten wurde. Kugelrohrdestillation bei 240 °C/0,3 mbar ergab 14,8 g α-Tocopherol. Das entspricht einer Ausbeute von 69 % der Theorie.

Beispiel 2

a) Herstellung einer (2RS, 6RS)-2,6,10-Trimethyl-1-undecyl-magnesiumchloridlösung

Zu 0,43 g (17,8 mmol) Magnesiumspänen, die durch Anätzen mit einem Jodkristall aktiviert worden waren, wurden, 4,08 g (17 mmol) (2RS, 6RS)-1-Chlor-2,6,10-trimethyl-undecan in 5 ml absolutem THF zugetropft. Das Gemisch wurde 2 h bei + 70 °C nachgerührt.

b) Umsetzung mit den Grignardreagentien

3,13 g (10 mmol) (2RS)-2,5,7,8-Tetramethyl-6-hydroxy-2-(2-bromethyl)-chroman wurden in 10 ml absolutem THF gelöst. Zu dieser Lösung wurden bei — 20 °C eine Lösung von Ethylmagnesiumbromid, die durch Umsetzen von 0,25 g (10,3 mmol) Magnesium in 3 ml THF mit einer Lösung von 1,1 g (10,0 mmol) Bromethan in 5 ml THF und lstündiges Rühren bei + 40 °C hergestellt worden war, zugetropft. Das erhaltene Reaktionsgemisch wurde 1 h bei — 20 °C nachgerührt und dann mit der in Beispiel 2a hergestellten (2RS, 6RS)-2,6,10-Trimethyl-1-undecylmagnesiumchloridlösung und anschließend mit 0,7 ml einer 0,5 molaren Lösung von Dilithiumtetrachlorocuprat in absolutem THF versetzt. Danach wurde 3 h bei — 20 °C und 16 h bei Raumtemperatur nachgerührt. Nach der Aufarbeitung des Reaktionsgemisches analog Beispiel 16 wurden durch Kugelrohrdestillation bei 220 °C/0,2 mbar 3,7 g α-Tocopherol isoliert. Das entspricht einer Ausbeute von 86 % der Theorie.

Beispiel 3

7,38 g (25 mmol) (2RS)-2,5,7,8-Tetramethyl-6-hydroxy-2-(2-brom-ethyl)-chroman wurden in 15 ml absolutem THF gelöst. Zu dieser Lösung wurden bei — 20 °C 15,8 ml einer 1,5 molaren Lösung von Methylmagnesiumbromid in THF (23,75 mmol) zugetropft und das Ganze 1 h bei — 20 °C nachgerührt.

Zu dem erhaltenen Reaktionsgemisch wurde bei — 20 °C eine Lösung von (2RS, 6RS)-2,6,10-Trimethyl-1-undecylmagnesiumchlorid, die analog Beispiel 1a aus 0,67 g (28 mmol) Magnesiumspänen und 6,40 g (27,5 mmol) (2RS, 6RS)-1-Chlor-2,6,10-trimethyl-undecan in 16 ml THF hergestellt wurde, addiert.

Anschließend wurde das Reaktionsgemisch bei — 20 °C mit 1,7 ml einer 0,5 molaren Lösung von Dilithiumtetrachlorocuprat in THF versetzt und das Ganze 3 h bei — 20 °C und 16 h bei Raumtemperatur nachgerührt.

Nach einer Aufarbeitung analog Beispiel 1c wurden durch destillative Reinigung im Kugelrohr bei 230 °C/0,2 mbar 6,9 g α-Tocopherol erhalten. Das entspricht einer Ausbeute von 64 % der Theorie.

Beispiel 4

7,38 g (25 mmol) (2RS)-2,5,7,8-Tetramethyl-6-hydroxy-2-(2-brom-ethyl)-chroman wurden in 20 ml THF gelöst. Hierdurch wurde bei — 20 °C eine Lösung von (2RS, 6RS)-2,6,10-Trimethyl-1-undecylmagnesiumchlorid, die analog Beispiel 1a aus 1,82 g (76 mmol) Magnesium und 17,44 g (75 mmol) (2RS, 6RS)-1-Chlor-2,6,10-Trimethylundecan in 38 ml THF hergestellt wurde, zugegeben.

Anschließend wurden bei — 20 °C 1,8 ml einer 0,5 molaren Lösung von Dilithiumtetrachlorocuprat in THF zugetropft. Das Reaktionsgemisch wurde 3 h bei — 20 °C und 15 h bei Raumtemperatur nachgerührt.

Das Gemisch wurde analog Beispiel 1c aufgearbeitet. Destillation im Kugelrohr bei 240 °C/0,3 mbar lieferte 8,7 g α-Tocopherol. Das entspricht einer Ausbeute von 81 % der Theorie.

Beispiel 5

7,38 g (25 mmol) (2S)-2,5,7,8-Tetramethyl-6-hydroxy-2-(2-brom-ethyl)-chroman wurden in 15 ml absolutem THF gelöst. Zu dieser Lösung wurden bei — 70 °C 15,8 ml einer 1,5-molaren Lösung von Methylmagnesiumbromid (23,75 mmol) in THF zugetropft. Es wurde 1 1/2 h bei — 70 °C nachgerührt und dann bei — 70 °C eine Lösung von (2RS, 6RS)-2,6,10-Trimethyl-1-undecylmagnesiumchlorid, die analog Beispiel 1a aus 0,92 g (38,5 mmol) Magnesium und 8,72 g (2RS, 6RS)-1-Chlor-2,6,10-Trimethyl-undecan in 20 ml THF hergestellt wurde, sowie 1,7 ml einer 0,5 molaren Lösung von Dilithiumtetrachlorocuprat in THF zugegeben. Das Reaktionsgemisch wurde 1 1/2 h bei — 70 °C, 2 h bei — 30 °C und 15 h bei Raumtemperatur nachgerührt.

Nach einer Aufarbeitung analog Beispiel 1c wurden durch Kugelrohrdestillation bei 220 °C/0,2 mbar

7,53 g (2R, 4'RS, 8'RS)-α-Tocopherol erhalten. Das entspricht einer Ausbeute von 70 % der Theorie.
Das (2R, 4'RS, 8'RS)-α-Tocopherol wurde in der üblichen Weise in das Acetat übergeführt.
$[\alpha]_D^{25} = + 2{,}54°$ (C = 5,2/Ethanol)

## Beispiel 6

7,38 g (25 mmol) (2S)-2,5,7,8-Tetramethyl-6-hydroxy-2-(2-brom-ethyl)-chroman wurden in 15 ml absolutem THF gelöst. Zu dieser Lösung wurden bei —20 °C 15,8 ml einer 1,5 molaren Lösung von Methylmagnesiumbromid (23,75 mmol) in THF zugetropft. Es wurde 1 h bei —20 °C nachgerührt. Dann wurde bei —20 °C eine Lösung von (2R, 6R)-2,6,10-Trimethyl-1-undecylmagnesiumchlorid, die analog Beispiel 1a aus 0,92 g (38,5 mmol) Magnesium und 8,72 g (2R, 6R)-1-Chlor-2,6,10-Trimethyl-undecan in 20 ml THF hergestellt wurde, sowie 1,9 ml einer 0,5 molaren Lösung von Dilithiumtetrachlorocuprat in THF zugegeben. Es wurde 3 h bei —20 °C und 15 h bei Raumtemperatur nachgerührt.

Nach der Aufarbeitung analog Beispiel 1c wurden durch Kugelrohrdestillation bei 230 °C/0,2 mbar 8,1 g (2R, 4'R, 8'R)-α-Tocopherol erhalten. Das entspricht einer Ausbeute von 75 % der Theorie.

Nach Überführung ins Acetat in der üblichen Weise zeigt das Produkt einen Drehwert von $[\alpha]_D^{25} = + 3{,}0°$ (C = 5,0 in Ethanol).

## Beispiel 7

6,26 g (20 mmol) (2RS)-2,5,7,8-Tetramethyl-6-hydroxy-2-(2-brom-ethyl)-chroman wurden in 20 ml absolutem THF gelöst. Zu dieser Lösung wurden bei —20 °C 16,2 ml einer 1,5 molaren Lösung von Vinylmagnesiumchlorid in THF zugegeben und noch 1 h bei —20 °C nachgerührt. Anschließend wurde bei —20 °C eine (2RS, 6RS)-1-Chlor-2,6,10-trimethyl-undecylmagnesiumchloridlösung, die durch Umsetzen einer Lösung von 0,86 g (35,8 mmol) Magnesium in 10 ml absolutem THF und einer Lösung von 8,16 g (34 mmol) (2RS, 6RS)-1-Chlor-2,6,10-trimethyl-undecan in 10 ml THF und zweistündiges Rühren bei + 70 °C hergestellt worden war, und danach 1,4 ml einer 0,5 molaren Lösung von Dilithiumtetrachlorocuprat in absolutem THF zugetropft. Das Reaktionsgemisch wurde noch 3 h bei —20 °C und 15 h bei Raumtemperatur nachgerührt. Nach einer Aufarbeitung analog Beispiel 1c wurden durch Destillation bei 210 °C/0,2 mbar 3,3 g α-Tocopherol isoliert. Das entspricht einer Ausbeute von 38 % der Theorie.

**Patentansprüche**

1. Verfahren zur Herstellung von α-Tocopherol der Formel I

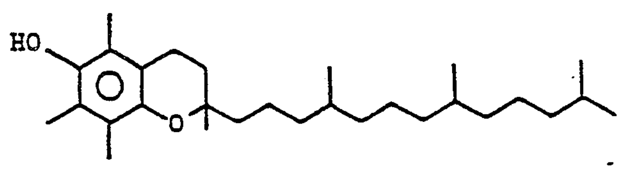

(I)

durch Umsetzung eines Chromanderivates mit einem $C_{14}$-Grignard-Reagens unter Katalyse mit einem Dialkalimetalltetrahalogencuprat nach Schlosser-Fouquet, dadurch gekennzeichnet, daß man

a) ein Chromanderivat der allgemeinen Formel II

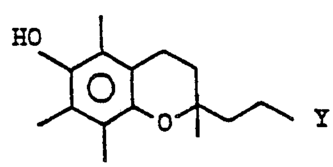

(II)

in der Y für eine der Fluchtgruppen -Br, -J, -p-Tosyl oder Benzolsulfonyl steht, verwendet und dieses bei Temperaturen von —70 bis 0 °C

b) zunächst mit der Lösung von 0,8 bis 1,1 Aquivalenten eines Grignard-Reagenses der allgemeinen Formel III

X—Mg—R (III),

in der X für Cl, Br oder J steht und R eine geradkettige oder verzweigte Alkylgruppe mit 1 bis 14 C-Atomen, bedeutet, und dann

c) mit der Lösung von 1,05 bis 2 Äquivalenten eines Grignard-Reagenses der allgemeinen Formel IIIa

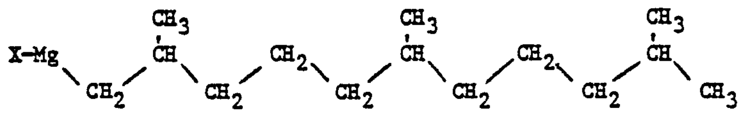

(IIIa)

in der X die obige Bedeutung aufweist, in einem etherischen Lösungsmittel und in Gegenwart der Lösung eines Dialkalimetalltetrahalogencuprats in einem etherischen Lösungsmittel umsetzt.

2. Verfahren gemäß Anspruch 1, dadurch gekennzeichnet, daß man zur Herstellung von (2R, 4'RS, 8'RS)-α-Tocopherol eine etherische Lösung von (2S)-2,5,7,8-Tetramethyl-6-hydroxy-2-(2'-brom-ethyl)-chroman in der Stufe c) mit der Lösung eines (2RS, 6RS)-2,6,10-Trimethyl-1-undecyl-magnesiumhalogenids umsetzt.

3. Verfahren gemäß Anspruch 1, dadurch gekennzeichnet, daß man zur Herstellung von (2R, 4'R, 8'R)-α-Tocopherol eine etherische Lösung von (2S)-2,5,7,8-Tetramethyl-6-hydroxy-2-(2'-brom-ethyl)-chroman in der Stufe c) mit der Lösung eines (2R, 6R)-2,6,10-Trimethyl-1-undecylmagnesiumhalogenids umsetzt.

4. Verfahren gemäß Anspruch 1, dadurch gekennzeichnet, daß man ein Chromanderivat der allgemeinen Formel II, in dem Y für -Br steht, einsetzt.

5. Verfahren gemäß Anspruch 1, dadurch gekennzeichnet, daß man das Chromanderivat der allgemeinen Formel II mit dem Grignard-Reagens der allgemeinen Formel III und mit dem Grignard-Reagens der allgemeinen Formel IIIa bei Temperaturen von —25 bis —15 °C umsetzt.

6. Verfahren gemäß Anspruch 1, dadurch gekennzeichnet, daß man das Chromanderivat der allgemeinen Formel II mit 0,85 bis 1,0 Äquivalenten eines Grignard-Reagenses der allgemeinen Formel III umsetzt.

7. Verfahren gemäß Anspruch 1, dadurch gekennzeichnet, daß man das Chromanderivat der allgemeinen Formel II mit einem Grignard-Reagens der allgemeinen Formel III umsetzt, in dem R eine Methylgruppe, eine Ethylgruppe oder den Rest

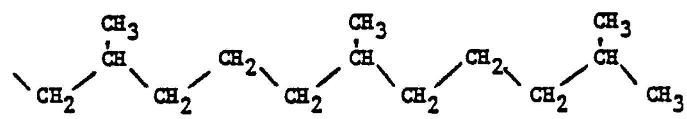

bedeutet.

8. Verfahren gemäß Anspruch 1, dadurch gekennzeichnet, daß man das Chromanderivat der allgemeinen Formel II zunächst mit dem Grignard-Reagens der allgemeinen Formel III und dann mit 1,2 bis 1,7 Äquivalenten des Grignard-Reagenses der allgemeinen Formel IIIa umsetzt.

## Claims

1. A process for the preparation of α-tocopherol of the formula I

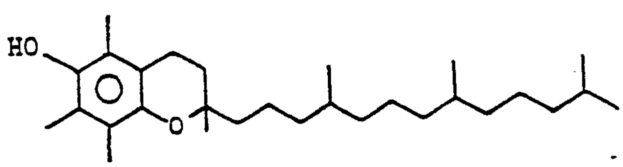

(I)

by reacting a chroman derivative with a $C_{14}$-Grignard reagent using a di-alkali metal tetrahalocuprate catalyst in a Schlosser-Fouquet reaction, wherein

a) a chroman derivative of the general formula II

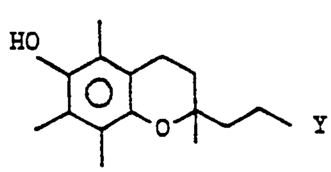

(II)

where Y is one of the leaving groups -Br, -I, -p-tosyl or benzenesulfonyl, is used and is reacted, at from —70 to 0 °C,

b) first with a solution of from 0.8 to 1.1 equivalents of a Grignard reagent of the general formula III

X—Mg—R (III),

where X is Cl, Br or I and R is straight-chain or branched alkyl of 1 to 14 carbon atoms, and then

c) with a solution of from 1.05 to 2 equivalents of a Grignard reagent of the general formula IIIa

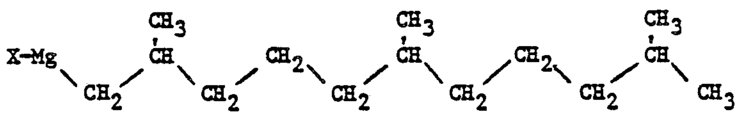

(IIIa)

where X has the above meanings, in an ether solvent and in the presence of a solution of a di-alkali metal tetrahalocuprate in an ether solvent.

2. A process as claimed in claim 1, wherein, to prepare (2R, 4'RS, 8'RS)-α-tocopherol, an ether solution of (2S)-2,5,7,8-tetramethyl-6-hydroxy-2-(2'-bromoethyl)-chroman is reacted, in step c), with a solution of a (2RS, 6RS)-2,6,10-trimethyl-1-undecyl-magnesium halide.

3. A process as claimed in claim 1, wherein, to prepare (2R, 4'R, 8'R)-α-tocopherol, an ether solution of (2S)-2,5,7,8-tetramethyl-6-hydroxy-2-(2'-bromoethyl)-chroman is reacted, in step c), with a solution of a (2R, 6R)-2,6,10-trimethyl-1-undecyl-magnesium halide.

4. A process as claimed in claim 1, wherein a chroman derivative of the general formula II, where Y is -Br, is used.

5. A process as claimed in claim 1, wherein the chroman derivative of the general formula II is reacted with the Grignard reagent of the general formula III and the Grignard reagent of the general formula IIIa at from —25 to —15 °C.

6. A process as claimed in claim 1, wherein the chroman derivative of the general formula II is reacted with 0.85 to 1.0 equivalent of a Grignard reagent of the general formula III.

7. A process as claimed in claim 1, wherein the chroman derivative of the general formula II is reacted with a Grignard reagent of the general formula III, where R is methyl, ethyl or the radical

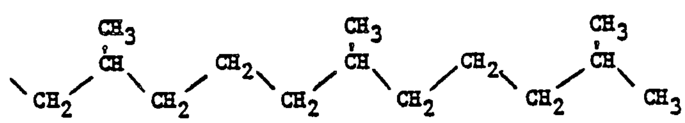

8. A process as claimed in claim 1, wherein the chroman derivative of the general formula II is reacted first with the Grignard reagent of the general formula III and then with 1.2 to 1.7 equivalents of the Grignard reagent of the general formula IIIa.

**Revendications**

1. Procédé de préparation d'α-tocophérol de formule I

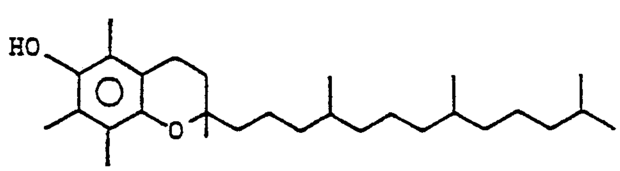

(I)

par réaction d'un dérivé du chromane avec un réactif de Grignard en $C_{14}$, catalysée par un tétrahalogénocuprate de di-(métal alcalin) suivant la méthode de Schlosser-Fouquet, caractérisé en ce qu'on utilise

a) un dérivé du chromane de formule générale

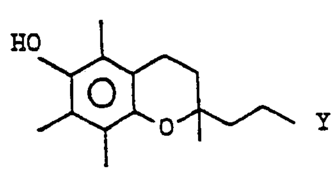

(II)

dans laquelle Y est mis pour l'un des groupes éliminables -Br, -I, -p-tosyle ou benzènesulfonyle, et on le fait réagir à une température de —70 à 0 °C

b) tout d'abord avec la solution de 0,8 à 1,1 équivalents d'un réactif de Grignard de formule générale III

$$X—Mg—R \qquad (III)$$

dans laquelle X est mis pour Cl, Br ou I et R représente un groupement alkyle à chaîne droite ou ramifiée à 1-14 atomes de C, puis

c) avec la solution de 1,05 à 2 équivalents d'un réactif de Grignard de formule générale IIIa

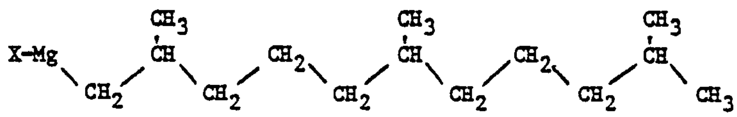

(IIIa)

dans laquelle X a la signification donnée ci-dessus, dans un solvant de la nature d'un éther et en présence de la solution d'un tétrahalogénocuprate de di-(métal alcalin) dans un solvant de la nature d'un éther.

2. Procédé selon la revendication 1, caractérisé en ce que pour la préparation de (2R, 4'RS, 8'RS)-α-tocophérol, on fait réagir une solution dans l'éther de (2S)-2,5,7,8-tétraméthyl-6-hydroxy-2-(2'-brométhyl)-chromane avec, dans la phase c), la solution d'un halogénure de (2RS, 6RS)-2,6,10-triméthyl-1-undécyl-magnésium.

3. Procédé selon la revendication 1, caractérisé en ce que pour la préparation de (2R, 4'R, 8'R)-α-tocophérol, on fait réagir une solution dans l'éther de (2S)-2,5,7,8-tétraméthyl-6-hydroxy-2-(2'-brométhyl)-chromane avec, dans la phase c), la solution d'un halogénure de (2R, 6R)-2,6,10-triméthyl-1-undécyl-magnésium.

4. Procédé selon la revendication 1, caractérisé en ce qu'on fait réagir un dérivé du chromane de formule générale II dans laquelle Y est mis pour -Br.

5. Procédé selon la revendication 1, caractérisé en ce qu'on fait réagir le dérivé du chromane de formule générale II avec le réactif de Grignard de formule générale III et avec le réactif de Grignard de formule générale IIIa à des températures de —25 à —15 °C.

6. Procédé selon la revendication 1, caractérisé en ce qu'on fait réagir le dérivé du chromane de formule générale II avec 0,85 à 1,0 équivalent d'un réactif de Grignard de formule générale III.

7. Procédé selon la revendication 1, caractérisé en ce qu'on fait réagir le dérivé du chromane de formule générale II avec un réactif de Grignard de formule III dans laquelle R représente un groupe méthyle, un groupe éthyle ou le radical

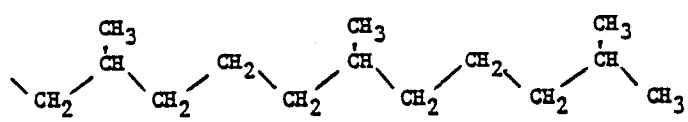

8. Procédé selon la revendication 1, caractérisé en ce qu'on fait réagir le dérivé du chromane de formule générale II, tout d'abord avec le réactif de Grignard de formule générale III, puis avec 1,2 à 1,7 équivalents du réactif de Grignard de formule générale IIIa.